# EUROPEAN PATENT APPLICATION

(11) **EP 3 202 405 A1**
(43) Date of publication of application: **09.08.2017**
(21) Application number: 15846533.6
(22) Date of filing: 02.10.2015
(51) Int. Cl.: A61K 31/702, A61K 9/14, A61K 9/19, A61K 47/36, A61P 1/02, A61P 1/10, A61P 3/06, A61P 37/02

(54) **OLIGOSACCHARIDE PREPARATION AND METHOD FOR MANUFACTURING SAME**

(30) Priority: 03.10.2014 JP 2014204799
(71) Applicant: Meiji Co., Ltd., Chuo-ku Tokyo 104-8306 (JP)
(72) Inventor: YOSHIOKA, Shin, Tokyo 104-0031 (JP)
(74) Representative: ABG Patentes, S.L.
(86) International application number: PCT/JP2015/078031
(87) International publication number: WO 2016/052721

(57) **Abstract**

An object of the present invention is to provide an oligosaccharide preparation having suppressed hygroscopicity and good storage stability while maintaining preferred physiological functions of oligosaccharides, and a method for producing the same. According to the present invention, there are provided a method for producing an oligosaccharide preparation comprising: step (a) of dissolving an oligosaccharide and acacia fiber in a solvent to give a solution and step (b) of spray-drying, vacuum-drying or freeze-drying the solution obtained in the step (a), and, optionally, step (c) of granulation, and an oligosaccharide preparation produced by the production method.

## Description

### CROSS-REFERENCE TO RELATED APPLICATION

The present application enjoys the benefit of priority from the prior Japanese Patent Application No. 2014-204799 filed on October 3, 2014, the entire disclosure of which is incorporated herein by reference.

### TECHNICAL FIELD

The present invention relates to an oligosaccharide preparation having low hygroscopicity and good storage stability and a method for producing the same.

### BACKGROUND ART

Oligosaccharides are carbohydrates comprising about 2 to 10 monosaccharides bound to each other. Fructooligosaccharide, raffinose, soybean oligosaccharide, galactooligosaccharide, xylooligosaccharide, lactosucrose, and the like are known as typical oligosaccharides.

In general, oligosaccharides are known to have physiological functions such as low cariogenicity, low calorie and intestine regulating function. More specifically, the effect of promoting intestinal Bifidobacterium growth, the intestine regulating function, the effect of promoting mineral absorption, the indigestibility (low calorie), the effect of improving the metabolism of lipids such as cholesterol, the immunoregulatory effect, the effect of suppressing blood glucose level elevation, and the like are known as the physiological functions of fructooligosaccharide.

Convenient ingestion of oligosaccharides having preferred physiological functions is required due to the rise in health trend. Therefore, not only forms of seasonings such as table sugar, but also various forms such as beverages, bread, desserts and confectioneries are found, as oligosaccharide-containing processed food products, in the market.

In general, oligosaccharides are readily hygroscopic. Therefore, there has been attempted suppression of hygroscopicity, for example, by a method for obtaining a high-purity crystal for a specific compound from an oligosaccharide (Patent Document 1) or a method for producing granules comprising a mixture of an oligosaccharide and sucrose (Patent Document 2).

### Reference List

### Patent Documents

Patent Document 1: JPB 2640577
Patent Document 2: JPA259100/1991

### SUMMARY OF INVENTION

A method for obtaining a low-hygroscopic, high-purity crystal from an oligosaccharide requires and a plurality of complicated steps such as a step of purification from a molasses solution, a separation/fractionation step and a crystallization step, and the resultant crystal would be expensive. Also, granules comprising a mixture of an oligosaccharide and sucrose would damage low calorie properties which are a preferred characteristic of oligosaccharides.

The present inventor reviewed whether hygroscopicity can be suppressed by adding low-calorie dietary fiber to an oligosaccharide, and found that a preparation containing an oligosaccharide (especially, inulin-free fructooligosaccharide, galactooligosaccharide or lactosucrose) and acacia fiber has low hygroscopicity. Also, the present inventor has found that the mass ratio of the oligosaccharide to the acacia fiber is defined within the range of from 30:70 to 56:44, thereby providing a preferred hygroscopicity suppressive effect. Further, the present inventor has found that the preparation can be produced through the steps of dissolving raw materials and then drying the resultant solution. The present invention is based on these findings.

An object of the present invention is to obtain an oligosaccharide preparation having suppressed hygroscopicity and good storage stability while maintaining preferred physiological functions of oligosaccharides.

The present invention provides the following inventions.
[1] A method for producing an oligosaccharide preparation comprising:
   step (a) of dissolving an oligosaccharide and acacia fiber in a solvent to give a solution, and
   step (b) of drying the solution obtained in the step (a) (hereinafter referred to as the "production method of the present invention" in some cases).
[2] The method according to (1), wherein the mass ratio of the oligosaccharide to the acacia fiber ranges from 30:70 to 56:44.
[3] The method according to [1] or [2], wherein the drying in the step (b) is spray drying, vacuum drying or freeze drying.
[4] The method according to any one of [1] to [3], which further comprises step (c) of granulation.
[5] The method according to any one of [1] to [4], wherein the oligosaccharide is one, two or more oligosaccharides selected from the group consisting of fructooligosaccharide, galactooligosaccharide and lactosucrose.
[6] The method according to [5], wherein the fructooligosaccharide is a fructooligosaccharide substantially free from inulin.
[7] An oligosaccharide preparation produced by the method according to any one of [1] to [6] (hereinafter referred to as the "oligosaccharide preparation of the present invention" in some cases).
[8] A dried oligosaccharide preparation comprising an oligosaccharide and acacia fiber.
[9] The preparation according to [8], which is a spray-dried preparation, a vacuum-dried preparation or a freeze-dried preparation.
[10] The preparation according to [8] or [9], wherein the properties of the preparation do not substantially change even after storage at 24°C for 2 days.
[11] A food or beverage product obtained by incorporating the oligosaccharide preparation according to any one of [7] to [10].
[12] A method for producing an oligosaccharide-containing food or beverage product, comprising the steps of: producing an oligosaccharide preparation by the method according to any one of [1] to [6]; and then incorporating the oligosaccharide preparation.

The present invention can provide an oligosaccharide preparation having low hygroscopicity and good storage stability.

### DETAILED DESCRIPTION OF THE INVENTION

As used herein, the term "oligosaccharide" means a carbohydrate comprising 2 to 10 monosaccharides bound to each other. Specifically, examples of the oligosaccharide include fructooligosaccharide, galactooligosaccharide, lactosucrose, raffinose, soybean oligosaccharide, xylooligosaccharide, lactulose, cellooligosaccharide and isomaltoligosaccharide. In the oligosaccharide preparation of the present invention, fructooligosaccharide, galactooligosaccharide and lactosucrose are preferably used.

A fructooligosaccharide which is substantially free from inulin is preferably selected as the fructooligosaccharide. Specifically, there are indicated fructooligosaccharides obtained by transferring fructose to sucrose through an enzyme reaction. More preferably, there are indicated inulin-free fructooligosaccharides comprising, as the main ingredient, 1-kestose (GF2), nystose (GF3) and fructofuranosylnystose (GF4) in which 1 to 3 molecules of fructose are bonded to sucrose. Specific examples of the fructooligosaccharides described above include MEIOLIGO P and MEIOLIGO G (both of which are trade names of Meiji Food Materia Co., Ltd.).

As used herein, the term "acacia fiber" means a food material which is sap collected from trees belonging to the genus Acacia, is an indigestible carbohydrate having a polysaccharide structure, and is referred to as "gum arabic" as a substance name. In order that the oligosaccharide preparation of the present invention has a good flavor and low hygroscopicity, acacia fiber obtained by a production method which does not comprise a decoloring step or a bleaching step is preferably selected as the acacia fiber. Examples of the acacia fiber include Fibregum P and Fibregum TAN (both of which are trade names of Nexira).

The production method of the present invention comprises:
step (a) of dissolving an oligosaccharide and acacia fiber in a solvent, and
step (b) of drying the solution obtained in the step (a), and, preferably, can further comprise:
   step (c) of granulating the oligosaccharide preparation obtained in the step (b). Also, the present invention provides an oligosaccharide preparation obtained by the production method of the present invention. Hereinafter, the steps (a), (b) and (c) will be described.

### Ste (a)

Step (a) is a step of dissolving an oligosaccharide and acacia fiber. More specifically, an oligosaccharide and acacia fiber are weighed so that the mass ratio of the oligosaccharide to acacia fiber ranges from 30:70 to 56:44, and a solvent is added to dissolve them therein, so that the solid content concentration (Brix) is adjusted to an appropriate value. The solvent may be added to dissolve them while warming and /or stirring. Water can suitably be used as the solvent. A component which can be added within a range in which the effects of the present invention would not be impaired may be added to the solvent. The solution obtained in this step is preferably adjusted preferably within the range of pH 5 to pH 7, more preferably within the range of pH 5.5 to pH 6.5. When the solution has a pH lower than the above-described range, such a pH is not desired since the oligosaccharide is easily hydrolyzed during the dissolution while warming in this step or during drying in the subsequent step (b). Also, when the solution has a pH higher than the above-described range, such a pH is not desired since coloring, which is presumed to be derived from the selected oligosaccharide, tends to occur. Incidentally, even if a powder obtained not by dissolving an oligosaccharide and acacia fiber, but merely by mixing the two components is granulated in step (c), an oligosaccharide preparation having suppressed hygroscopicity cannot be obtained.

The mass ratio of an oligosaccharide to acacia fiber used in the production method of the present invention and the content ratio of an oligosaccharide to acacia fiber in the oligosaccharide preparation of the present invention can be defined within the range of from 30:70 to 56:44, preferably within the range of from 30:70 to 55.3:44.7, more preferably within the range of from 40:60 to 56:44 or 40:60 to 55.3:44.7, especially preferably within the range of from 45:55 to 56:44 or 45:55 to 55.3:44.7, for suppression of moisture absorption, deliquescence and solidification and for better exertion of the functions of the oligosaccharide. When the ratio of the acacia fiber is higher in the mass ratio and content ratio outside the above-described range, no particular problem occurs from the viewpoint of suppressing the hygroscopicity. However, a higher ratio of the oligosaccharide to the acacia fiber is desired, from the viewpoint of taking advantage of the functions of the oligosaccharide.

Additives, other than the oligosaccharide and the acacia fiber, may also be incorporated in the oligosaccharide preparation of the present invention within a range in which the effect of the present invention would not be impaired. Examples of the additives include emulsifiers, thickeners, colorants, perfumes, preservatives, microbicides, brighteners, nutrition enhancers, excipients, binders, disintegrants and lubricants. Some or all of these additives can be dissolved together when the oligosaccharide and the acacia fiber are dissolved in step (a).

### Ste (b)

Step (b) is a step of drying the solution obtained in step (a). Examples of a drying means include spray drying, vacuum drying and freeze drying, and the spray drying step is preferred. Drying conditions are not especially limited. The drying treatment in step (b) can provide the oligosaccharide preparation having suppressed hygroscopicity according to the present invention. The oligosaccharide preparation obtained by spray drying or vacuum drying has the property of suppressed hygroscopicity, but tends to have rather low solubility in liquids. Also, the oligosaccharide preparation obtained by freeze drying has excellent solubility in liquids while having the property of suppressed hygroscopicity, but is bulky and tends to have low operation aptitude for mixing operation or the like and/or low production aptitude for tableted products.

### Step (c)

Step (c) is a step of further granulating the oligosaccharide preparation obtained in step (b). While the granulation method is not especially limited, a fluidized bed granulation method is preferred. The solubility, powder flying, and tableting aptitude of the oligosaccharide preparation obtained in the step (b) can be improved by adding this step to the step (b). The steps (b) and (c) can also be carried out concurrently by some facilities.

The oligosaccharide preparation of the present invention comprises at least an oligosaccharide and acacia fiber. Also, the oligosaccharide preparation of the present invention is one obtained by drying an oligosaccharide and acacia fiber which are dissolved in a solvent. Thus, it can be said that the oligosaccharide preparation of the present invention is a dried preparation (preferably, a spray-dried preparation, a vacuum-dried preparation or a freeze-dried preparation). That is, the present invention provides a dried oligosaccharide preparation comprising an oligosaccharide and acacia fiber. The phrase "the oligosaccharide preparation of the present invention," as used herein, also includes the dried oligosaccharide preparations described above.

The oligosaccharide preparation of the present invention can be applied to known food and beverage products and medicaments. Specifically, the present invention provides a food or beverage product and a medicament each obtained by incorporating the oligosaccharide preparation of the present invention. The food or beverage product and medicament can be produced by adding or incorporating the oligosaccharide preparation of the present invention to or in other raw materials.

The oligosaccharide preparation of the present invention has a tinge of sweetness, but less affects the taste of the product to which it is applied. Further, the oligosaccharide preparation has the effect of increasing the body feeling when the product to which it is applied is a food product. The oligosaccharide preparation has the effect of masking an unpleasant taste when the product is a food/beverage product. No matter which product is produced, the hygroscopicity is suppressed, and thus the product is easy to handle. Especially, the oligosaccharide preparation of the present invention has excellent tableting aptitude, and thus can be preferably utilized as a base for various tableted products. A tableted product comprising a high content of an oligosaccharide can be obtained using a direct tableting method by merely adding small amount of an emulsifier to the oligosaccharide preparation of the present invention. Therefore, tablets are indicated as preferred products to which the oligosaccharide preparation is applied.

As indicated in the Examples which will be described later, the oligosaccharide preparation of the present invention has excellent low moisture absorption characteristics. For example, the oligosaccharide preparation, when allowed to stand and stored at 24°C for 2 days, has the characteristic that the properties of the preparation do not substantially change, as compared with the preparation at the time of beginning of storage. Also, the preparation, when allowed to stand and stored under the conditions: a temperature of 24 °C and a humidity of 50%, has the characteristic that it does not solidify until after 120 minutes, as compared with the preparation at the time of beginning of storage.

According to the present invention, there is provided an oligosaccharide preparation having low hygroscopicity and good storage stability. The oligosaccharide preparation of the present invention can ensure easy packaging at the time of sale, by virtue of its low hygroscopicity.

Also, the oligosaccharide preparation of the present invention itself has a tinge of sweetness, but, when it is used in various food and beverage products and the like, the taste of the final products is hardly affected (i.e., no excessive sweetness). Thus, the oligosaccharide preparation of the present invention can be used in a wide range of products. For example, the effect of enhancing the body feeling of taste and the effect of masking an unpleasant taste are obtained in some kinds of final products.

Further, the granulated preparation of the present invention has excellent tableting aptitude. Therefore, the preparation can be preferably utilized as a base for various tableted products. In general, oligosaccharides have bad tableting aptitude due to their high hygroscopicity, and are regarded as being difficult to be incorporated, in a high proportion of 30% or more, in a tableted product. However, a small amount of emulsifier is merely added to the oligosaccharide preparation of the present invention, so that a tableted product comprising a high content of an oligosaccharide can be obtained by the direct tableting method.

The present invention provides the following inventions.
[11] An oligosaccharide preparation produced by a method comprising:
   step (a) of dissolving components comprising an oligosaccharide and acacia fiber in a mass ratio ranging from 30:70 to 55.3:44.7 in a solvent to give a solution, and
   (b) spray-drying, vacuum-drying or freeze-drying the solution obtained in the step (a).
[12] An oligosaccharide preparation produced by a method comprising:
   step (c) of granulating the oligosaccharide preparation according to [11].
[13] The oligosaccharide preparation according to [11] or [12], wherein the oligosaccharide is at least one oligosaccharide selected from fructooligosaccharide, galactooligosaccharide and lactosucrose.
[14] The oligosaccharide preparation according to [13], wherein the fructooligosaccharide is a fructooligosaccharide free from inulin.
[15] A food or beverage product comprising the oligosaccharide preparation according to any one of [11] to [14].

### EXAMPLES

The present invention will be described in detail by way of the following Examples, but is not limited to these Examples.

### Example 1

Water was added to an oligosaccharide and dietary fiber mixed in ratios as indicated in Table 1 to prepare aqueous solutions having a Brix of 65. The aqueous solutions were each adjusted using sodium carbonate so as to have a pH of approximately 6. The aqueous solutions in the respective test sections after preparation were vacuum-dried to prepare oligosaccharide preparations. Five (5) g of the respective resultant preparations were weighed in a petri dish, allowed to stand at room temperature (24 °C) for 2 days, and then visually observed in terms of moisture absorbing state. The results are indicated in Table 1.

Fructooligosaccharide (MEIOLIGO P (liquid), Meiji Food Materia Co., Ltd.) was used as the oligosaccharide, and acacia fiber (Fibregum P, Nexira) was used as the dietary fiber. The fructooligosaccharide used is oligosaccharides comprising, as the main ingredient, 1-kestose (GF2), nystose (GF3) or fructofuranosylnystose (GF4) in which 1 to 3 molecules of fructose are bonded to sucrose, having monosaccharide and sucrose contents of 5% or less in the solid content and free from inulin. Also, the acacia fiber used is non-decolored dietary fiber obtained from the species *Acacia senegal.*
[Table 1]

**Table 1: Ratio between oligosaccharide and dietary fiber incorporated**

| Raw material | Fructooligosaccharide (MEIOLIGO P (liquid)) | Acacia fiber (Fibregum P) | State of preparation after storage at room temperature (24 °C) for 2 days |
|---|---|---|---|
| Test Section 1-A | 30g | 70g | Almost the same as preparation after vacuum drying |
| Test Section 1-B | 40g | 60g | Almost the same as preparation after vacuum drying |
| Test Section 1-C | 50g | 50g | Almost the same as preparation after vacuum drying |
| Test Section 1-D | 60g | 40g | The surface solidified through moisture absorption. |
| Test Section 1-E | 70g | 30g | The surface solidified through moisture absorption. |

From Table 1, oligosaccharide preparations having low hygroscopicity could be obtained in the range of the mass ratio of the oligosaccharide (fructooligosaccharide) to the dietary fiber (acacia fiber) of from 30:70 to 50:50.

### Example 2

Twenty (20) ml of water was added to 10 g of an oligosaccharide (fructooligosaccharide, trade name: MEIOLIGO P (powder), Meiji Food Materia Co., Ltd.) to dissolve the oligosaccharide with stirring, thereby preparing an aqueous oligosaccharide solution. One hundred (100) ml of water was added to 10 g of acacia fiber (trade name: Fibregum P, Nexira) to dissolve the acacia fiber with stirring in a warm bathtub at 80 °C, thereby preparing an aqueous dietary fiber solution. After mixing the aqueous oligosaccharide solution and the aqueous dietary fiber solution, the mixture was freeze-dried (device name: Vacuum Freeze Dryer Model FD-1, shelf temperature: 35 °C), thereby obtaining an oligosaccharide preparation. The oligosaccharide preparation was bulky, but was low hygroscopic and non-deliquescent.

### Example 3

Water was added to 210 g of an oligosaccharide (fructooligosaccharide, trade name: MEIOLIGO P (liquid), Meiji Food Materia Co., Ltd.) and 170 g of dietary fiber to dissolve the oligosaccharide and dietary fiber at 80 °C with stirring, thereby preparing an aqueous solution having a Brix of 40. As the dietary fiber, acacia fiber (trade name: Fibregum P, Nexira), polydextrin (trade name: Litesse Powder, Danisco Japan Co., Ltd.) and indigestible dextrin (trade name: Fibersol-2, Matsutani Chemical Industry Co., Ltd.) were used. The aqueous solutions were each adjusted using sodium carbonate so as to have a pH of approximately 6. The aqueous solutions in the respective test sections after preparation were spray-dried (device name: Model SD LT-8, inlet temperature: 130 °C and outlet temperature: 90 °C), and then granulated (device name: FLOW COATER Model FLO-5), thereby preparing oligosaccharide preparations. As a binder used in the granulation step, an aqueous solution of 10 mass % of the spray dried product was used. Five (5) g of the respective preparations obtained in the granulation step were weighed in a petri dish, and allowed to stand in an environment at a temperature of 24 °C and a humidity of 50% or at a temperature of 30 °C and a humidity of 60% for 0 minute to 240 minutes. The preparations were visually observed in terms of mass increment and appearance. The results are indicated in Table 2.
[Table 2]

**Table 2: Ratio between oligosaccharide and various dietary fibers incorporated and change in properties and the like**

| Raw material | | Test Section 2-A | Test Section 2-B | Test Section 2-C |
|---|---|---|---|---|
| Oligosaccharide | Fructooligosaccharide | 210 g | 210 g | 210 g |
| Dietary fiber | Acacia fiber | 170g | - | - |
| | Polydextrin | - | 170g | - |
| | Indigestible dextrin | - | - | 170g |
| Still standing condition | Still standing time | Mass increment (g) | Mass increment (g) | Mass increment (g) |
| Still standing at 24°C· 50% humidity | 0 min. | 0 | 0 | 0 |
| | 30 min. | 0.110 | 0.127 | 0.100 |
| | 60 min. | 0.149 | 0.202 | 0.141 |
| | 90 min. | 0.178 | 0.241 | 0.171 |
| | 120 min. | 0.215 | 0.310 | 0.219 |
| | 240 min. | 0.253 | 0.362 | 0.263 |
| | Visual observation result | The product did not solidify until after 120 min. Even after 240 min., it did not deliquesce or solidify at all, and became easy to handle simply by addition of light vibration. | The product began to solidify after 90 min., and had a sticky appearance after 240 min. | The surface began to solidify after 120 min., and the product contained moisture and had a hard appearance after 240 min. |
| Still standing at 30°C· humidity 60% | 0 min. | 0 | 0 | 0 |
| | 30 min. | 0.103 | 0.160 | 0.126 |
| | 60 min. | 0.193 | 0.280 | 0.229 |
| | 90 min. | 0.228 | 0.321 | 0.266 |
| | 120 min. | 0.286 | 0.376 | 0.332 |
| | 240 min. | 0.343 | 0.406 | 0.389 |
| | Visual observation result | The surface began to solidify after 120 min., but, even after 240 min, only the surface solidified. | The periphery began to solidify after 30 min., and the product had a sticky appearance after 120 min. | The product began to solidify as a whole after 90 min., and, after 240 min., it contained moisture, had a glossy appearance, and completely solidified. |

From the results in Table 2, under either of the conditions: a temperature of 24 °C and a humidity of 50%; and a temperature of 30 °C and a humidity of 60%, the oligosaccharide preparation (Test Section 2-A) comprising fructooligosaccharide and acacia fiber in a mass ratio of 55.3:44.7 (210 g: 170 g) was most difficult to solidify. Also, the oligosaccharide preparation in Test Section 2-A described above did not deliquesce or solidify at all even after the elapse of 240 minutes from the beginning of still standing, and became easy to handle simply by addition of light vibration.

Incidentally, when the structures and physical properties of the raw material (provided that powdery fructooligosaccharide was used) and oligosaccharide preparation in Test Section 2-A were analyzed, thermal behaviors, which are seen in amorphous materials, were observed in the raw material fructooligosaccharide and physical mixture of fructooligosaccharide and acacia fiber by differential scanning calorimetry. However, such behaviors disappeared in the oligosaccharide preparation. From this result, it was suggested that an oligosaccharide preparation with a structure and physical properties different from those of the raw material was produced by the drying step.

### Example 4

Two (2) parts by mass of an emulsifier (trade name: Poem TR-FB, RIKEN VITAMIN CO., LTD.) was added to 98 parts by mass of the oligosaccharide preparation (fructooligosaccharide:acacia fiber=21:17) in Test Section 2-A obtained in Example 3, and well mixed. Thereafter, a tablet (a) having a diameter (ϕ) of 8 mm to 9 mm and a mass of 250 mg to 310 mg was obtained using a tableting machine (Model: FY-MS2-30, manufactured by FUJI YAKUHIN KIKAI Co., Ltd.). No trouble such as capping occurred in the production of the tablet (a), and a tableted product having good appearance and low hygroscopicity could be obtained. Also, the resultant tablet (a) had good taste quality with a tinge of sweetness of the oligosaccharide.

### Comparative Example 1

A powder mixture comprising 2 kg of an oligosaccharide (fructooligosaccharide, trade name: MEIOLIGO P (powder), Meiji Food Materia Co., Ltd.) and 2 kg of acacia fiber (trade name: Fibregum P, Nexira) mixed well was prepared. The powder mixture was granulated (device name: Dalton Corporation kneading/high-speed stirring granulator Model: SPGJ-25TG and fluidized bed dryer Model: MDG-380), thereby preparing an oligosaccharide preparation. Water was used as a binder for the granulation step.

The oligosaccharide preparation prepared appeared to have slightly improved hygroscopicity as compared with the oligosaccharide alone, but was highly hygroscopic and sticky just with a touch, as with conventional oligosaccharides.

### Example 5

As oligosaccharides, 50 g of fructooligosaccharide (trade name: MEIOLIGO P (liquid), Meiji Food Materia Co., Ltd.), galactooligosaccharide (trade name: Cup Oligo P (powder), Nissin Sugar Co., Ltd.) and lactosucrose (trade name: Oligo-no-Okage LS-55P, ENSUIKO SUGAR REFINING CO., LTD.), respectively, were mixed with 50 g of acacia fiber (trade name: Fibregum P, Nexira), and water was added to dissolve them, thereby preparing aqueous solutions having a Brix of 65. The aqueous solutions were each adjusted using sodium carbonate so as to have a pH of approximately 6. The aqueous solutions in the respective test sections after preparation were spray-dried (device name: Model SD LT-8, inlet temperature: 130 °C and outlet temperature: 90 °C), thereby preparing oligosaccharide preparations.

Five (5) g of the respective preparations obtained were weighed in a petri dish, and allowed to stand in an environment at a temperature of 24 °C and a humidity of 50% for 0 hour to 72 hours or in an environment at a temperature of 27 °C and a humidity of 65% for 0 hour to 18 hours. The preparations were visually observed in terms of mass increment and appearance. The results are indicated in Table 3.
[Table 3]

**Table 3: Ratio between various oligosaccharides and dietary fiber incorporated and change of properties and the like**

| Raw material | | Test Section 3-A | Test Section 3-B | Test Section 3-C |
|---|---|---|---|---|
| Oligosaccharide | Fructooligosaccharide | 50g | - | - |
| | Galactooligosaccharide | - | 50g | - |
| | Lactosucrose | - | - | 50g |
| Dietary fiber | Acacia fiber | 50g | 50g | 50g |

| Still standing condition | Still standing time | Mass increment (g) | Mass increment (g) | Mass increment (g) |
|---|---|---|---|---|
| Still standing at 24 °C· 50% humidity | 0 hr. | 0 | 0 | 0 |
| | 72 hr. | 0.65 | 0.7 | 0.75 |
| | Visual observation result | The surface solidified, but the product did not deliquesce. | The surface solidified, but the product did not deliquesce. | The surface solidified, but the product did not deliquesce. |
| Still standing at 27°C· humidity 65% | 0 min. | 0 | 0 | 0 |
| | 30 min. | 0.060 | 0.058 | 0.055 |
| | 60 min. | 0.098 | 0.162 | 0.120 |
| | 90 min. | 0.180 | 0.234 | 0.230 |
| | 120 min. | 0.286 | 0.317 | 0.324 |
| | 150 min. | 0.320 | 0.340 | 0.347 |
| | 18 hr. | 0.460 | 0.400 | 0.473 |
| | Visual observation result | The surface began to slightly harden after 120 min., but, even after 18 hr., only the surface solidified. The product had easy-to-disintegrate property. | The surface began to slightly harden after 120 min., but, even after 18 hr., only the surface solidified. The product had easy-to-disintegrate property. | The surface began to slightly harden after 120 min., but, even after 18 hr., only the surface solidified. The product had easy-to-disintegrate property. |

From the results indicated in Table 3, the oligosaccharide preparations comprising acacia fiber and each of the oligosaccharides (fructooligosaccharide, galactooligosaccharide and lactosucrose) indicated in Table 3 had low hygroscopicity and excellent storage stability without completely deliquescing even after the elapse of 72 hours from the beginning of still standing. Incidentally, when 5 g of fructooligosaccharide used in Example 5 was allowed to stand at a temperature of 27 °C and a humidity of 65%, it absorbed moisture and deliquesced after the elapse of 1 hour.

### Example 6

According to the formulations indicated in Table 4, chocolates were produced through normal steps (mixing process, refiner process, conching process, molding process).
[Table 4]

**Table 4: Formulation of chocolate**

| | Example 6-1 | Example 6-2 |
|---|---|---|
| Oligosaccharide preparation of the present invention (Test Section 2-A in Example 3) | 25 parts by mass | 40 parts by mass |
| Cacao mass | 65 parts by mass | 20 parts by mass |
| Cocoa butter | 5 parts by mass | 20 parts by mass |
| Cocoa powder | 5 parts by mass | - |
| Whole milk powder | - | 20 parts by mass |
| Emulsifier | 1 part by mass | 0.5 part by mass |
| Perfume | 0.2 part by mass | 0.1 part by mass |

Normally, when sugar to be incorporated in chocolate is replaced with a fructooligosaccharide powder (for example, MEIOLIGO P powder) to produce chocolate, it is difficult to produce chocolate in accordance with normal steps due to high viscosity of chocolate base in the midway of the production. The oligosaccharide preparation of the present invention could be used to produce chocolate without causing the production problem described above.

### Example 7

According to the formulations indicated in Table 5, baked confectioneries comprising the oligosaccharide preparation of the present invention were produced. Margarine and the oligosaccharide preparation of the present invention or sugar were mixed, and then the mixture was emulsified by adding egg. Thereafter, flour was further mixed to prepare dough. After shaping of the dough, the dough was baked at 180 °C for 11 minutes, thereby obtaining a cookie.
[Table 5]

**Table 5: Formulation of cookie**

| | Control | | Example 7 | |
|---|---|---|---|---|
| | Mass (g) | Incorporation rate (%) | Mass (g) | Incorporation rate (%) |
| Oligosaccharide preparation of the present invention (Test Section 2-A in Example 3) | 0 | 0 | 36 | 12 |
| Sugar | 60 | 20 | 24 | 8 |
| Margarine | 90 | 30 | 90 | 30 |
| Egg | 15 | 5 | 15 | 5 |
| Flour | 135 | 45 | 135 | 45 |
| Total | 300 | 100 | 300 | 88 |

The cookie produced by incorporating the oligosaccharide preparation of the present invention has good appearance with rather yellowish color as compared with a control product, and had a tough, crunchy texture as compared with the control product.

### Example 8

According to the formulations indicated in Table 6, powder beverages comprising the oligosaccharide preparation of the present invention were produced by a known granulation method (the method described in JPB 26512/1975).
[Table 6]

**Table 6: Formulation of powder beverage**

| | Control | Example 8-1 | Example 8-2 |
|---|---|---|---|
| Oligosaccharide preparation of the present invention (Test Section 2-A in Example 3) | 0 | 15.0 | 15.0 |
| Indigestible dextrin | 59.0 | 51.5 | 44.0 |
| Sugar | 12.0 | 4.5 | 12.0 |
| Vegetable oil/fat | 0.3 | | |
| Emulsifier | 0.2 | | |
| Vitamin powder | 0.6 | | |
| Calcium | 0.4 | | |
| Ferric pyrophosphate | 0.05 | | |
| Sweetener | 0.3 | | |
| Colorant | 0.4 | | |
| Perfume | 2.0 | | |
| Vegetable·fruit powder | 4.0 | | |
| Skimmed milk powder | 5.0 | | |
| Modified starch | 10.0 | | |
| Acidulant | 2.0 | | |
| Total (parts by mass) | 96.25 | | |

In comparison between the bulk specific gravities of the resultant powder beverages, no difference was found between the control and the products comprising the oligosaccharide preparation of the present invention. Also, the particle size distributions of the respective powder beverages were confirmed by means of a micro-type electromagnetic vibration sieve. The results are indicated in Table 7.
[Table 7]

**Table 7: Result of sieving**

| Proportion (%) | Control | Example 8-1 | Example 8-2 |
|---|---|---|---|
| On 14 mesh | 0.1 | 0.0 | 0.0 |
| On 26 mesh | 6.0 | 6.9 | 6.5 |
| On 48 mesh | 13.6 | 39.5 | 40.6 |
| On 60 mesh | 4.1 | 3.6 | 4.2 |
| Passing through 60 mesh | 76.2 | 49.9 | 48.7 |

As indicated in Table 7, the control powder beverage contained a lot of a fraction with a fine particle size passing through 60 mesh, whereas the powder beverages comprising the oligosaccharide preparation of the present invention contained a lot of a fraction with a particle size larger than that of the control. Therefore, the oligosaccharide preparation of the present invention had good granulation aptitude in the production of the powder beverages.

Twenty (20) g of the resultant powder beverage was charged into 200 mL of water at 23 °C, and stirred by means of a spatula for 20 seconds, thereby preparing a beverage. The beverage comprising the oligosaccharide preparation of the present invention had no drying smell, and had a mild and rich flavor as compared with the control beverage. Also, when the beverage prepared was filtered through a sieve (22 mesh) to observe a residue, the beverage comprising the oligosaccharide preparation of the present invention had good solubility since it was less lumpy than the control beverage.

### Example 9

A powder beverage comprising the oligosaccharide preparation of the present invention was produced by mixing the components indicated in Table 8.
[Table 8]

**Table 8: Formulation of powder beverage**

| | Example 9 |
|---|---|
| Oligosaccharide preparation of the present invention (Test Section 2-A in Example 3) | 30 |
| Cassis polyphenol AC10 powder (manufactured by Meiji Food Materia Co., Ltd.) | 14 |
| Sweetener (glucose) | 40 |
| Sweetener (sucralose) | 1 |
| Acidulant (citric acid) | 12 |
| Perfume | 3 |
| Total (parts by mass) | 100 |

In 180 ml of clear water, 3.5 g of the resultant powder beverage was dissolved to prepare a beverage. This powder beverage was rapidly dissolved merely by lightly stirring. The beverage comprising the oligosaccharide preparation of the present invention had a refreshing flavor while having the body feeling.

## Claims

1. A method for producing an oligosaccharide preparation comprising:
step (a) of dissolving an oligosaccharide and acacia fiber in a solvent to give a solution, and
step (b) of drying the solution obtained in the step (a).

2. The method according to claim 1, wherein the mass ratio of the oligosaccharide to the acacia fiber ranges from 30:70 to 56:44.

3. The method according to claim 1 or 2, wherein the drying in the step (b) is spray drying, vacuum drying or freeze drying.

4. The method according to any one of claims 1 to 3, which further comprises step (c) of granulation.

5. The method according to any one of claims 1 to 4, wherein the oligosaccharide is one, two or more oligosaccharides selected from the group consisting of fructooligosaccharide, galactooligosaccharide and lactosucrose.

6. The method according to claim 5, wherein the fructooligosaccharide is a fructooligosaccharide substantially free from inulin.

7. An oligosaccharide preparation produced by the method according to any one of claims 1 to 6.

8. A dried oligosaccharide preparation comprising an oligosaccharide and acacia fiber.

9. The preparation according to claim 8, which is a spray-dried preparation, a vacuum-dried preparation or a freeze-dried preparation.

10. The preparation according to claim 8 or 9, wherein the properties of the preparation do not substantially change even after storage at 24°C for 2 days.

11. A food or beverage product obtained by incorporating the oligosaccharide preparation according to any one of claims 7 to 10.

12. A method for producing an oligosaccharide-containing food or beverage product, comprising the steps of: producing an oligosaccharide preparation by the method according to any one of claims 1 to 6; and then incorporating the oligosaccharide preparation.
